(19) **Europäisches Patentamt / European Patent Office / Office européen des brevets**

(11) **EP 4 563 088 B1**

(12) **EUROPEAN PATENT SPECIFICATION**

(45) Date of publication and mention of the grant of the patent:
**01.04.2026 Bulletin 2026/14**

(21) Application number: **23383235.1**

(22) Date of filing: **30.11.2023**

(51) International Patent Classification (IPC):
***A61B 5/372*** (2021.01)    ***A61B 5/00*** (2006.01)

(52) Cooperative Patent Classification (CPC):
**A61B 5/372; A61B 5/4064; A61B 5/7264;**
A61B 5/7267

(54) **METHOD FOR PROCESSING AN ELECTROENCEPHALOGRAPHY DATA FILE**

VERFAHREN ZUR VERARBEITUNG EINER DATEI FÜR ELEKTROENZEPHALOGRAFIEDATEN

PROCÉDÉ DE TRAITEMENT D'UN FICHIER DE DONNÉES D'ÉLECTROENCÉPHALOGRAPHIE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(43) Date of publication of application:
**04.06.2025 Bulletin 2025/23**

(73) Proprietor: **Fundación Universidad Francisco de Vitoria**
**28223 Pozuelo de Alarcón - Madrid (ES)**

(72) Inventors:
- **GARCÍA TEJEDOR, Álvaro José**
**28223 Pozuelo de Alarcón, Madrid (ES)**
- **MAITÍN LÓPEZ, Ana María**
**28223 Pozuelo de Alarcón, Madrid (ES)**
- **ÁLVAREZ DE TOLEDO LLOBERA, Inés**
**28223 Pozuelo de Alarcón, Madrid (ES)**
- **ROMERO MUÑOZ, Juan Pablo**
**28223 Pozuelo de Alarcón, Madrid (ES)**

(74) Representative: **TRBL Intellectual Property**
**Plaza de Castilla 3, 7°A**
**28046 Madrid (ES)**

(56) References cited:
- **LU JIANCHAO ET AL: "PearNet: A Pearson Correlation-based Graph Attention Network for Sleep Stage Recognition", 2022 IEEE 9TH INTERNATIONAL CONFERENCE ON DATA SCIENCE AND ADVANCED ANALYTICS (DSAA), IEEE, 13 October 2022 (2022-10-13), pages 1 - 8, XP034289110, DOI: 10.1109/ DSAA54385.2022.10032354**
- **DOMINIK KLEPL ET AL: "Adaptive Gated Graph Convolutional Network for Explainable Diagnosis of Alzheimer's Disease using EEG Data", ARXIV.ORG, CORNELL UNIVERSITY LIBRARY, 201 OLIN LIBRARY CORNELL UNIVERSITY ITHACA, NY 14853, 10 August 2023 (2023-08-10), XP091581062**

## EP 4 563 088 B1

**Description**

### TECHNICAL FIELD

This invention belongs to the field of methods for signal processing.

### STATE OF THE ART

An electroencephalogram ("EEG") is a known tool that measures and records the electrical activity of a person's brain in order to evaluate cerebral activity. To do so, several electrodes are arranged in a person's head, so that the electric activity of the brain is obtained. The device then amplifies the signals and stores data corresponding to this electrical activity of a person's brain.

Each electrode is focused on a specific region of the brain. However, the raw data obtained by the EEG does not always provide the necessary information to the physician.

Document "PearNet: A Pearson Correlation-based Graph Attention Network for Sleep Stage Recognition", by Jianchao, Lu et al. (doi.org/10.48550/arXiv.2209.13645) proposes a Pearson correlation-based graph attention network as a solution to this problem. Graph nodes are generated based on the spatial-temporal features extracted by a hierarchical feature extraction method, and then the graph structure is learned adaptively to build node connections.

Document "Adaptive Gated Graph Convolutional Network for Explainable Diagnosis of Alzheimer's Disease Using EEG Data", by Klepl, Dominik, et al. (DOI: 10.1109/TNSRE.2023.3321634) proposes a model that achieves high accuracy in both eyes-closed and eyes-open conditions, indicating the stability of learned representations and demonstrates that the proposed AGGCN model generates consistent explanations of its predictions that might be relevant for further study of AD-related alterations of brain networks.

The present invention provides a method for processing this signal.

### DESCRIPTION OF THE INVENTION

The invention provides an alternative solution for this problem by means of a method according to claim 1. Preferred embodiments of the invention are defined in dependent claims.

Unless otherwise defined, all terms (including technical and scientific terms) used herein are to be interpreted as is customary in the art. It will be further understood that terms in common usage should also be interpreted as is customary in the relevant art and not in an idealised or overly formal sense unless expressly so defined herein.

In this text, the term "comprises" and its derivations (such as "comprising", etc.) should not be understood in an excluding sense, that is, these terms should not be interpreted as excluding the possibility that what is described and defined may include further elements, steps, etc.

In a first inventive aspect, the invention provides a method for processing an electroencephalography data file, the method comprising several steps a. to i.:

In step a., an electroencephalography device containing electrodes produces a data file containing a plurality of channels, each channel containing data of one electrode for a temporal range, the temporal range being the same for every channel and lasting a plurality of seconds.

This first step is performed by any type of electroencephalography device as available by the skilled person. This electroencephalography device comprises a plurality of electrodes, which may be arranged on the head of a patient or implanted in his brain. Each electrode is configured to perceive an electric signal coming from the patient's brain which is duly transformed to create the electroencephalography data file. This data file comprises a plurality of channels, where each channel contains the data from one electrode. The information of the data file is extended during a temporal range. All the electrodes produce their data during this temporal range, so the electroencephalography data file contains a number of channels, each channel containing data from the corresponding electrode in the common temporal range.

In step, b. the processor is used to retrieve the data file and extract at least one temporal window from the temporal range, each temporal window lasting a subrange of seconds.

The processor retrieves the data file produced by the electroencephalography device. This data file may be retrieved from a server, or received by electronic communication means or any other way available for the skilled person.

The processor then extracts at least one temporal window from the temporal range. This can be done, for example, by dividing the temporal range in a plurality of temporal windows. However, a portion of this temporal range may be disregarded, so that the sum of all the temporal windows may be smaller than the total temporal range. It is also possible that a single temporal window (that may cover the whole temporal range or not) is chosen. Hence, the processor will have the information from all the channels for the first temporal window, the information from all the channels for the second temporal window, and so on.

In step c., the processor is used to generate one graph for one of the temporal windows, using the data of all the channels in the corresponding temporal window, wherein the graph contains nodes and edges, each edge connecting a pair of nodes, wherein each node refers to an electrode associated to one channel and wherein each edge contains a connectivity value obtained from the data for the corresponding pair of nodes joined by the edge.

The complete data file includes all the channels in the whole temporal range. In step b., some temporal windows were extracted. In this step c., for each temporal window, a graph is created. Each graph contains the information of all the channels but only for the corresponding temporal window (as will be seen later, there will be one graph per temporal window). This information is divided into nodes and edges. Each edge connects a pair of nodes. The nodes will just have the information of the position of the channel. The edges will contain the information received from the channels (for this temporal window) in the shape of a connectivity value, which is obtained from the data of the two nodes of the pair of nodes connected by the corresponding edge: if an edge connects nodes 1 and 2, this edge will have a connectivity value obtained from the data of nodes 1 and 2.

In step d., the processor is used to define a connectivity vector for each node, wherein the connectivity vector has a size of Nx1, N being the number of nodes, and wherein each element of the connectivity vector equals to the connectivity value of an edge for the pair of nodes which have a connectivity value equal or higher than a connectivity threshold, and equals to zero for the nodes which have a connectivity value lower than a connectivity threshold.

The graphs are now converted into vectors and matrices so that they can be fed by an algorithm. Firstly, a connectivity vector is defined for each node (i.e., for each channel, i.e., for each electrode). Each connectivity vector will therefore have a size with Nx1, since it contains, for each node, the connectivity values with all the nodes (including the one with itself). Element 1 of the connectivity vector of node 1 will contain the connectivity value of the node 1 with itself. Element 2 of the connectivity vector will contain the connectivity value of the node 1 with the node 2, and so on. However, if a connectivity value is below a threshold, the value of the corresponding element will be zero.

In step e., the processor is used to define an adjacency matrix containing an adjacency vector for each node, wherein the value of each element of an adjacency vector corresponds to the value of the same element of the equivalent connectivity vector, but adjusted to 1 if this element is greater than zero.

An adjacency matrix is defined to identify which are the "active edges" in the system. Hence, the values of the elements of the adjacency matrix will be either 1 or 0: 1 for all the nodes where the connectivity value was higher than the threshold and 0 for all the nodes where the connectivity value was lower than the threshold.

In step f., the set of connectivity vectors and the adjacency matrix are introduced as an input in a graph attention network run by the processor, wherein the graph attention network is configured to apply a transformation over each connectivity vector using the adjacency matrix as an input, thus obtaining a final output value for each connectivity vector.

Steps c. to e. have created one connectivity vector per node and an adjacency matrix. In this step f., the connectivity vectors are introduced in a graph attention network together with the adjacency matrix. The graph attention network will process each connectivity vector, using the adjacency matrix, until arriving at an output value for each connectivity vector. Hence, at the end, the output values can be arranged in a final output vector.

In step g., each final output value is arranged in a temporal output vector, so that a temporal output vector is obtained for all the nodes for the temporal window.

In step h., steps c. to g. are repeated for each temporal window, thus obtaining a number of temporal output vectors which corresponds to the number of temporal windows.

Steps c. to g. were aimed to obtain a temporal output vector for each temporal window. This temporal output vector contained one element for each node. Then, this step h. repeats the steps c. to g. for all the temporal windows (the number of temporal windows is equal or greater than 1). The method, at this step h., provides a number of temporal output vectors equal to the number of temporal windows.

In step i., a final output vector is obtained by using the graph attention network to calculate the mean value of the temporal output vectors.

At the end, it is useful to have a single final output vector, which represents a final output value for each node. Since the method, at step h., provided a number of temporal output vectors corresponding to the number of temporal windows, the average value of these temporal output vectors is obtained, by summing them and dividing into the number of vectors, to achieve a final output vector, which provides a final value for each node.

This method obtains a processed signal with a final output vector, which provides a numeric value for each node, thus providing a measurable amount for each node.

The graph attention network has been previously trained by performing steps b. to j. with a dataset which comprises data files of electroencephalography devices and by correcting the output of the graph attention network.

The dataset includes data files of electroencephalography devices. The training supervisor knows the output value that should be produced by the graph attention network and corrects the output value. Hence, the weight values and coefficients of the graph attention network are adjusted so that they may work with the real data.

In some particular embodiments, the subrange of seconds is 2 seconds.

The subrange of seconds may virtually be any number of seconds, but this amount has been proved to be efficient and to provide good results.

In some particular embodiments, the step of obtaining the connectivity value for each edge in step c. is performed by dividing the covariance of the data of the pair of nodes of the corresponding edge by the product of the standard deviations of the data of each of the two nodes of the pair of nodes.

To define the connectivity value of each edge (i.e., the connectivity value between the two nodes connected by this edge), the Pearson Correlation is used: each edge connects two nodes, and each node represents one channel. The edge uses the information of channel 1 (corresponding to node 1) and the information of channel 2 (corresponding to node 2). To calculate the connectivity value of the edge, the covariance of the two channels 1 and 2 is calculated, and also the standard deviation of the information of channel 1 and of the information of channel 2, and then the covariance is divided by the product of the two standard deviations.

In some particular embodiments, the connectivity threshold is comprised between 0.6 and 0.7.

The threshold may be set in any place between 0 and 1, but this amount has been proved to be efficient and to provide good results.

The graph attention network comprises a first graph attention layer and a second graph attention layer, wherein

the first graph attention layer is configured to receive the adjacency matrix and the connectivity vectors as inputs and comprises two attention mechanisms, each attention mechanism being configured to provide an output vector of four values for each connectivity vector, so that the first graph attention layer is configured to provide, for each connectivity vector, a first output vector of eight values obtained by the concatenation of the two output vectors of four values produced by each attention mechanism;

the second graph attention layer is configured to receive the first output vector and the adjacency matrix as inputs, and comprises four attention mechanisms, each attention mechanism being configured to provide an output value, so that the second graph attention layer is configured to provide, for each first output vector, a single mean value obtained by calculating the mean value of the four output values produced by each attention mechanism; and

the graph attention network is further configured to apply a sigmoid transformation to the mean value, thus obtaining the final output value associated to the connectivity vector for the corresponding temporal window.

This specific network does not depend on the number of nodes (i.e., does not depend on the number of channels, and hence, on the number of electrodes), since the results will be a single final value per node, regardless the number of nodes.

The graph attention network has two layers. The first layer receives each connectivity vector one by one, and produces a vector of eight values as an output. This vector is the result of some operations performed by the attention mechanisms. This total output vector is fed to the second layer, which provides a single mean value, by calculating the mean value of the four values produced by the attention mechanisms (each attention mechanism produces one value, so there are four values, one per attention mechanism). Hence, the graph attention mechanism produces a single final value per connectivity vector per temporal window. A final operation of sigmoid transformation is used to provide a value in the format of a probabilistic result (contained between 0 and 1).

The step of training uses the output of the second graph attention layer and calculates a loss function as the binary cross entropy with logit loss.

This loss function helps this model to produce a better outcome.

Each attention mechanism of the first graph attention layer is configured to apply the following transformation:

$$h_i^{(l+1)} = \sigma\left(\sum_{j \in N(i)} \alpha_{ij}^{(l)} W h_j^{(l)}\right)$$

wherein

$h_i^{(l+1)}$ is the representation of node i obtained as the output of the attention layer;

$h_j^{(l)}$ is the representation of the nodes which are neighbouring node i;

**W** is the weight matrix for feature transformation and has a size of Nx4, N being the number of nodes;

$\sigma$ is the activation function and equals to Exponential Linear Unit (alpha: 1,0)

$\alpha_{ij}^{(l)}$ is the attention coefficient of the node i with node j,

$$\alpha_{ij}^{(l)} = softmax_j\Big(\,\sigma_a\big(a^T * [\,Wh_i \,\|\, Wh_j\,]\big)\Big),$$

$\sigma_a$ is the attention coefficient activation function and is LeakyReLU (step = 0,2);
$Wh_i \| Wh_j$ is the concatenation of the transformed representations of node i and node j;
$a^T$ is the attention weight matrix and has a size of 1x8.

Each attention mechanism of the second graph attention layer is configured to apply the following transformation:

$$h_i^{(l+1)} = \sigma\left(\sum_{j\in N(i)} \alpha_{ij}^{(l)} Wh_j^{(l)}\right)$$

wherein

$h_i^{(l+1)}$ is the representation of node i obtained as the output of the attention layer;

$h_j^{(l)}$ is the representation of the nodes which are neighbouring node i;
$W$ is the weight matrix and has a size of 8x1;
$\sigma$ is the activation function and equals to identity function

$\alpha_{ij}^{(l)}$ is the attention coefficient of the node i with node j,

$$\alpha_{ij}^{(l)} = softmax_j\Big(\,\sigma_a\big(a^T * [\,Wh_i \,\|\, Wh_j\,]\big)\Big),$$

$\sigma_a$ is the attention coefficient activation function and is LeakyReLU (step = 0,2);
$Wh_i \| Wh_j$ is the concatenation of the transformed representations of node i and node j;
$a^T$ is the attention weight matrix and has a size of 1x2.

These values have proved to provide good results in the analysis of EEG signals.

## BRIEF DESCRIPTION OF THE DRAWINGS

To complete the description and in order to provide for a better understanding of the invention, a set of drawings is provided. Said drawings form an integral part of the description and illustrate an embodiment of the invention, which should not be interpreted as restricting the scope of the invention, but just as an example of how the invention can be carried out. The drawings comprise the following figures:

Figure 1 shows the elements of a device used in a method according to the invention.

Figure 2 shows the extraction of temporal windows, as a step of a method according to the invention.

Figure 3 shows further steps of a method according to the invention, that will be repeated for each temporal window.

Figure 4 shows more steps of a method according to the invention, that will be repeated for each temporal window.

Figure 5 shows the structure of a graph attention network used in some steps of a method according to the invention.

In these figures, the following reference numbers are used

1   EEG Device
2   Electrodes
3   Head of a person
4   Electronic circuits of the EEG Device
5   Main processor

6       Temporal window
7       Nodes
8       Edge
9       Connectivity matrix
10      Adjacency matrix
11      First graph attention layer
12      Second graph attention layer
13      Attention mechanism of the first graph attention layer
14      Output vector of the attention mechanism of the first graph attention layer
15      First output vector
16      Attention mechanism of the second graph attention layer
17      Output value of the attention mechanism of the second graph attention layer
18      Single mean value
19      Final output value
20      Temporal output vector

## DETAILED DESCRIPTION OF THE INVENTION

The example embodiments are described in sufficient detail to enable those of ordinary skill in the art to embody and implement the systems and processes herein described. It is important to understand that embodiments can be provided in many alternate forms and should not be construed as limited to the examples set forth herein.

Accordingly, while embodiment can be modified in various ways and take on various alternative forms, specific embodiments thereof are shown in the drawings and described in detail below as examples. There is no intent to limit to the particular forms disclosed. On the contrary, all modifications, equivalents, and alternatives falling within the scope of the appended claims should be included. Elements of the example embodiments are consistently denoted by the same reference numerals throughout the drawings and detailed description where appropriate.

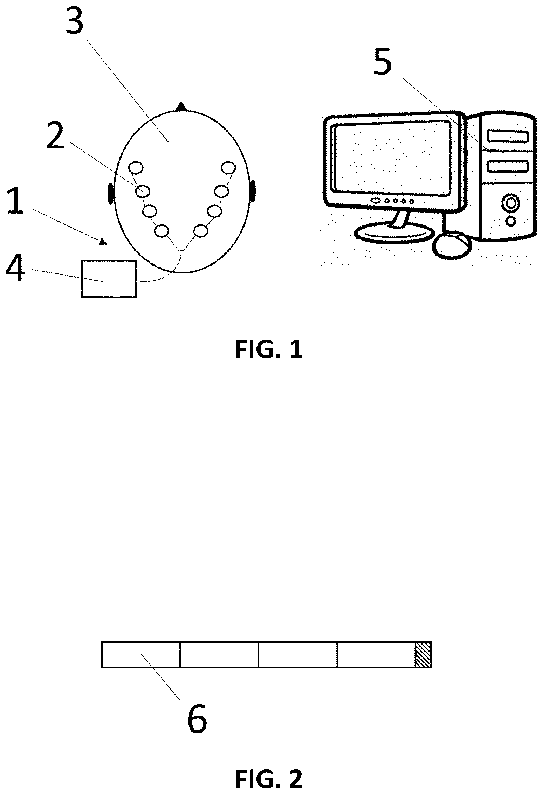

Figure 1 shows the elements of a device configured to implement a method according to the invention.

The device comprises an electroencephalography (EEG) device 1. This EEG device comprises a plurality of electrodes 2 (in this case, there are only 8 electrodes for the sake of simplicity, but this invention may be applied to any number of electrodes), intended to be arranged on the head of a person 3. This EEG device also comprises some electronic circuits 4 aimed to prepare the information received from the electrodes and produce a data file containing a plurality of channels. Each channel contains data of one electrode for a temporal range. The temporal range is the time during which the electric signals are received by the electrodes. Hence, this temporal range is the same for every channel and lasts a plurality of seconds.

Each channel contains the data from one electrode 2. All the electrodes 2 produce their data during this temporal range, so the data file contains a number of channels, each channel containing data from the corresponding electrode in the common temporal range.

Once the EEG device 1 has produced the data file, the main processor 5 retrieves this data file (this can be done by a downloading from a server, or a direct retrieving from a local host). The first step that the main processor 5 does to the data file is dividing this temporal range into temporal windows. This can be done in many different ways: an exact division of the whole temporal range into a number of temporal windows (the number of temporal windows can be 1 or can be another natural number) or just an extraction of one or more temporal windows, disregarding the rest of the data.

Figure 2 shows how, in this case, the temporal range of 8.4 seconds is divided into four temporal windows 6 of 2 seconds, and the remainder 0.4 seconds are disregarded. The data file contains information of all the channels for each temporal window. Hence, after this step, four different temporal windows 6 are extracted from the whole temporal range, one for each temporal window. Each temporal window 6 contains information for all the channels.

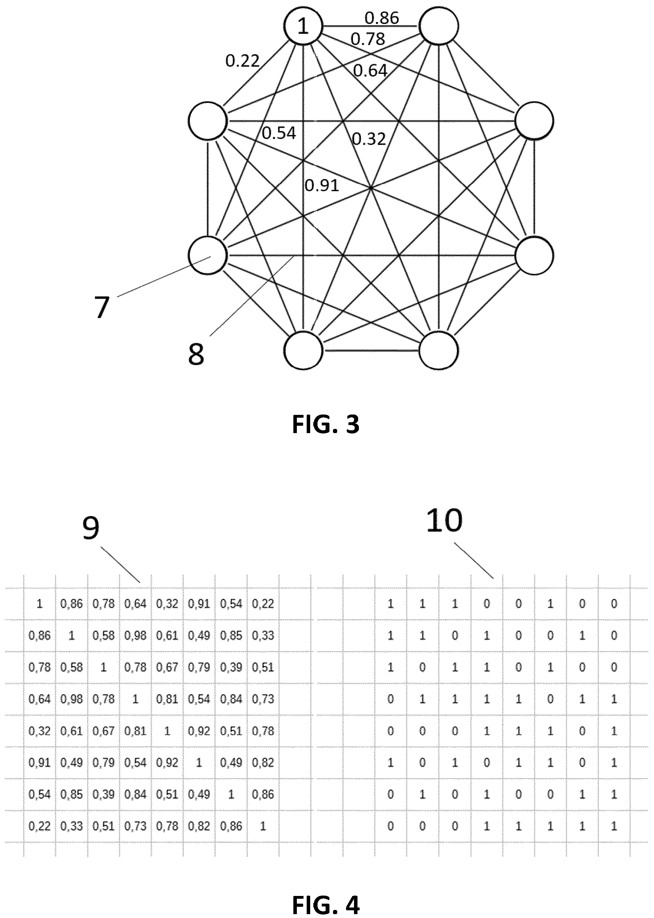

Figure 3 shows some steps of the method, that will be repeated for each temporal window. Firstly, a graph is generated for the first temporal window, using the data of all the channels in the corresponding temporal window. In the present example, the data file contains 8 channels, since there were 8 electrodes in the EEG device. The graph contains nodes 7 (numbered from 1 to 8) and edges 8. Each edge 8 connects a pair of nodes 7. Each node 7 refers to one channel (i.e., to the data of one electrode) and each edge 8, which join a pair of nodes 7, refers to the relation between the channels of the nodes 7 joint by said edge 8. Each edge 8 is assigned a connectivity value obtained from the data for the corresponding pair of nodes joined by the edge in the present temporal window.

The connectivity value of each edge is obtained by dividing the covariance of the data of the pair of nodes of the corresponding edge by the product of the standard deviations of the data of each of the two nodes of the pair of nodes:

$$C_{x,y} = \frac{\sigma_{xy}}{\sigma_x \sigma_y},$$

Where x and y are the nodes connected by the edge for which the connectivity value is being calculated.

As a consequence, each node will have a number of connectivity values which will be equal to the number of channels (in this case, 8). For example, node number 1 will have a connectivity value for the correlation between node 1 and itself (which will be equal to 1), then a connectivity value for the correlation between node 1 and node 2 (edge 1-2), etc.

These connectivity values for each node are used to create a connectivity vector for each node. But the values of the element of the connectivity vector are not just a transposition of the connectivity values: the connectivity values are included in the connectivity vector only if such values are higher than a threshold. Otherwise, a zero is placed instead.

For example, as seen in this figure, values for node 1 are: 1, 0.86, 0.78, 0.64, 0.32, 0.91, 0.54, 0.22. The threshold value is established in 0.7. Hence, the connectivity vector for node 1 will be (1, 0.86, 0.78, 0, 0, 0.91, 0, 0).

This structure is followed for every node, so that the method will produce a connectivity vector for each node for the present temporal window. Hence, a connectivity matrix is formed with all the connectivity vectors. This connectivity matrix will have a shape of NxN, N being the number of nodes of the graph.

Figure 4 shows an example of this connectivity matrix 9 which incorporates all the connectivity vectors. Then, and adjacency matrix 10 is created using the data of the connectivity vectors. This adjacency matrix represents the active connection between nodes: if a value is not zero in the connectivity matrix, it will correspond to a value of 1 in the adjacency matrix. If a value is zero, it will remain zero. Hence, the adjacency matrix will have only 0 and 1 values: 1 for the connections which produced a connectivity value higher than the threshold and 0 for the rest of the connections.

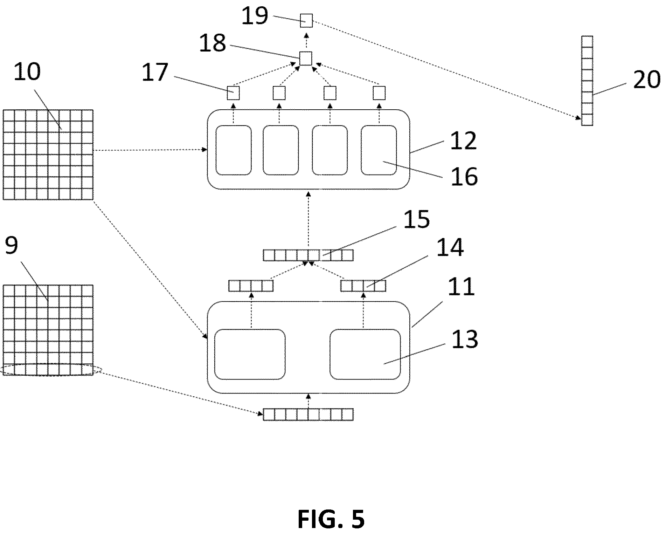

Figure 5 shows the structure of a graph attention network run by the processor. The set of connectivity vectors and the adjacency matrix are introduced as an input in this graph attention network.

The graph attention network comprises a first graph attention layer 11 and a second graph attention layer 12.

The first graph attention layer 11 is configured to receive the adjacency matrix 10 and the connectivity vectors of the connectivity matrix 9 as inputs and comprises two attention mechanisms 13. Each attention mechanism 13 is configured to provide an output vector 14 of four values for each connectivity vector, so that the first graph attention layer 11 is configured to provide, for each connectivity vector, a first output vector 15 of eight values (regardless the number of nodes) obtained by the concatenation of the two output vectors 14 of four values produced by each attention mechanism 13.

The transformation applied by each attention mechanism of this first graph attention layer is the following

$$h_i^{(l+1)} = \sigma\left( \sum_{j \in N(i)} \alpha_{ij}^{(l)} W h_j^{(l)} \right)$$

wherein

$h_i^{(l+1)}$ is the representation of node i obtained as the output of the attention layer;

$h_j^{(l)}$ is the representation of the nodes which are neighbouring node i;

W is the weight matrix for feature transformation and has a size of Nx4, N being the number of nodes;

σ is the activation function and equals to Exponential Linear Unit (alpha: 1,0)

$\alpha_{ij}^{(l)}$ is the attention coefficient of the node i with node j,

$$\alpha_{ij}^{(l)} = softmax_j\left( \sigma_a\left( a^T * [\, W h_i \,\|\, W h_j \,] \right) \right),$$

$\sigma_a$ is the attention coefficient activation function and is LeakyReLU (step = 0,2);

$W h_i \| W h_j$ is the concatenation of the transformed representations of node i and node j;

$a^T$ is the attention weight matrix and has a size of 1x8.

The second graph attention layer 12 is configured to receive the first output vector 15 and the adjacency matrix 10 as inputs, and comprises four attention mechanisms 16. Each attention mechanism is configured to provide an output value 17, so that the second graph attention layer is configured to provide, for each first output vector 15, a single mean value 18 is obtained by calculating the mean value of the four output values produced by each attention mechanism.

The transformation applied by each attention mechanism of this second graph attention layer is the following

$$h_i^{(l+1)} = \sigma\left(\sum_{j \in N(i)} \alpha_{ij}^{(l)} W h_j^{(l)}\right)$$

wherein

$h_i^{(l+1)}$ is the representation of node i obtained as the output of the attention layer;

$h_j^{(l)}$ is the representation of the nodes which are neighbouring node i;

**W** is the weight matrix and has a size of 8x1;

σ is the activation function and equals to identity function;

$\alpha_{ij}^{(l)}$ is the attention coefficient of the node i with node j,

$$\alpha_{ij}^{(l)} = softmax_j\left(\sigma_a\left(a^T * [Wh_i \| Wh_j]\right)\right),$$

$\sigma_a$ is the attention coefficient activation function and is LeakyReLU (step = 0,2);

$Wh_i \| Wh_j$ is the concatenation of the transformed representations of node i and node j;

$a^T$ is the attention weight matrix and has a size of 1x2.

The single mean value 18 is obtained by calculating the mean value of the four output values 17 produced by each attention mechanism 16. Once that this single mean value 18 has been produced by the second attention layer 12, the graph attention network applies a sigmoid transformation to the mean value, thus obtaining the final output value 19 associated to the connectivity vector for the corresponding temporal window.

Each final output value 19 is arranged in a temporal output vector 20, so that a temporal output vector is obtained for all the nodes for the temporal window.

All these steps are aimed to obtain the temporal output vector, but these steps only concern one temporal window.

All these steps are then repeated for the rest of the temporal windows. Once that all the steps are performed for all temporal windows, the network will have a number of temporal output vectors corresponding to the number of temporal windows.

Finally, the average value of these temporal output vectors is obtained, by summing them and dividing into the number of vectors, to achieve a final output vector, which provides a final value for each node.

This specific network does not depend on the number of nodes (i.e., does not depend on the number of channels, and hence, on the number of electrodes), since the results will be a single final value per node, regardless the number of nodes.

The graph attention network has been previously trained with a dataset of data files of electroencephalography devices, and using the output of the second graph attention layer and calculating a loss function as the binary cross entropy with logit loss.

**Claims**

1.    A method for processing an electroencephalography data file, the method comprising the steps of:

a. an electroencephalography device (1) containing electrodes (2) produces a data file containing a plurality of channels, each channel containing data of one electrode for a temporal range, the temporal range being the same for every channel and lasting a plurality of seconds;

b. using a processor (5) to retrieve the data file and extract at least one temporal window of the temporal range, each temporal window lasting a subrange of seconds;

c. using the processor to generate one graph for one of the temporal windows, using the data of all the channels in the corresponding temporal window, wherein the graph contains nodes (7) and edges (8), each edge (8) connecting a pair of nodes (7), wherein each node (7) refers to an electrode associated to one channel and wherein each edge (8) contains a connectivity value obtained from the data for the corresponding pair of nodes (7) joined by the edge (8);

**characterised in that** the method further comprises the steps of:

d. using the processor (5) to define a connectivity vector for each node, wherein the connectivity vector has a size of Nx1, N being the number of nodes, and wherein each element of the connectivity vector equals to the connectivity value of an edge for the pair of nodes which have a connectivity value equal or higher than a connectivity threshold, and equals to zero for the nodes which have a connectivity value lower than a connectivity threshold;

e. using the processor to define an adjacency matrix (10) containing an adjacency vector for each node, wherein the value of each element of an adjacency vector corresponds to the value of the same element of the equivalent connectivity vector, but adjusted to 1 if this element is greater than zero;

f. introducing the set of connectivity vectors and the adjacency matrix (10) as an input in a graph attention network run by the processor, wherein the graph attention network is configured to apply a transformation over each connectivity vector using the adjacency matrix (10) as an input, thus obtaining a final output value for each connectivity vector;

g. arranging each final output value in a temporal output vector, so that a temporal output vector is obtained for all the nodes for the temporal window;

h. repeating the steps c. to g. for each temporal window, thus obtaining a number of temporal output vectors (20) which corresponds to the number of temporal windows; and

i. obtaining a final output vector by using the graph attention network to calculate the mean value of the temporal output vectors (20),

and wherein

the graph attention network has been previously trained by performing steps b. to i. with a dataset which comprises data files of electroencephalography devices and by correcting the output of the graph attention network,

the graph attention network comprises a first graph attention layer and a second graph attention layer, wherein the first graph attention layer is configured to receive the adjacency matrix and the connectivity vectors as inputs and comprises two attention mechanisms (13), each attention mechanism being configured to provide an output vector (14) of four values for each connectivity vector, so that the first graph attention layer is configured to provide, for each connectivity vector, a first output vector (15) of eight values obtained by the concatenation of the two output vectors (14) of four values produced by each attention mechanism (13);

the second graph attention layer is configured to receive the first output vector (15) and the adjacency matrix as inputs, and comprises four attention mechanisms (16), each attention mechanism being configured to provide an output value (17), so that the second graph attention layer is configured to provide, for each first output vector, a single mean value (18) obtained by calculating the mean value of the four output values (17) produced by each attention mechanism (16);

the graph attention network is further configured to apply a sigmoid transformation to the mean value, thus obtaining the final output value (19) associated to the connectivity vector for the corresponding temporal window;

the step of training the graph attention network uses the output of the second graph attention layer and calculates a loss function as the binary cross entropy with logit loss;

each attention mechanism (13) of the first graph attention layer is configured to apply the following transformation:

$$h_i^{(l+1)} = \sigma \left( \sum_{j \in N(i)} \alpha_{ij}^{(l)} W h_j^{(l)} \right)$$

wherein

$h_i^{(l+1)}$ is the representation of node i obtained as the output of the attention layer;

$h_j^{(l)}$ is the representation of the nodes which are neighbouring node i;

**W** is the weight matrix for feature transformation and has a size of Nx4, N being the number of nodes;

σ is the activation function and equals to Exponential Linear Unit (alpha: 1,0) $\alpha_{ij}^{(l)}$ is the attention coefficient of the node i with node j,

$$\alpha_{ij}^{(l)} = softmax_j\left(\sigma_a\left(a^T * [\,Wh_i \,\|\, Wh_j\,]\right)\right),$$

$\sigma_a$ is the attention coefficient activation function and is LeakyReLU (step = 0,2);
$Wh_i \,\|\, Wh_j$ is the concatenation of the transformed representations of node i and node j;
$a^T$ is the attention weight matrix and has a size of 1x8.

each attention mechanism (16) of the second graph attention layer is configured to apply the following transformation:

$$h_i^{(l+1)} = \sigma\left(\sum_{j\in N(i)} \alpha_{ij}^{(l)} Wh_j^{(l)}\right)$$

wherein

$h_i^{(l+1)}$ is the representation of node i obtained as the output of the attention layer;

$h_j^{(l)}$ is the representation of the nodes which are neighbouring node i;

$W$ is the weight matrix and has a size of 8x1;
σ is the activation function and equals to identity function;

$\alpha_{ij}^{(l)}$ is the attention coefficient of the node i with node j,

$$\alpha_{ij}^{(l)} = softmax_j\left(\sigma_a\left(a^T * [\,Wh_i \,\|\, Wh_j\,]\right)\right),$$

$\sigma_a$ is the attention coefficient activation function and is LeakyReLU (step = 0,2);
$Wh_i \,\|\, Wh_j$ is the concatenation of the transformed representations of node i and node j;

$a^T$ is the attention weight matrix and has a size of 1x2.

2. Method according to any of the preceding claims, wherein the subrange of seconds is 2 seconds.

3. Method according to any of the preceding claims, wherein the step of obtaining the connectivity value for each edge in step c. is performed by dividing the covariance of the data of the pair of nodes of the corresponding edge by the product of the standard deviations of the data of each of the two nodes of the pair of nodes.

4. Method according to any of the preceding claims, wherein the connectivity threshold is comprised between 0.6 and 0.7.

**Patentansprüche**

1. Verfahren zum Verarbeiten einer Datei für Elektroenzephalographiedaten, wobei das Verfahren die folgenden Schritte umfasst:

a. Erzeugen, durch eine Elektroenzephalographievorrichtung (1), die Elektroden (2) enthält, einer Datendatei, die eine Vielzahl von Kanälen enthält, wobei jeder Kanal Daten einer Elektrode für einen Zeitbereich enthält, wobei der Zeitbereich für jeden Kanal der gleiche ist und eine Vielzahl von Sekunden dauert;
b. Verwenden eines Prozessors (5), um die Datendatei abzurufen und mindestens ein Zeitfenster des Zeitbereichs zu extrahieren, wobei jedes Zeitfenster einen Teilbereich von Sekunden dauert;

c. Verwenden des Prozessors, um einen Graphen für eines der Zeitfenster zu generieren, unter Verwendung der Daten aller Kanäle in dem entsprechenden Zeitfenster, wobei der Graph Knoten (7) und Kanten (8) enthält, wobei jede Kante (8) ein Paar von Knoten (7) verbindet, wobei jeder Knoten (7) sich auf eine einem Kanal zugeordnete Elektrode bezieht und wobei jede Kante (8) einen Konnektivitätswert enthält, der aus den Daten für das entsprechende Paar von Knoten (7), die durch die Kante (8) vereint sind, erhalten wird;

**dadurch gekennzeichnet, dass** das Verfahren ferner die folgenden Schritte umfasst:

d. Verwenden des Prozessors (5), um einen Konnektivitätsvektor für jeden Knoten zu definieren, wobei der Konnektivitätsvektor eine Größe von Nx1 hat, wobei N die Anzahl der Knoten ist, und wobei jedes Element des Konnektivitätsvektors gleich dem Konnektivitätswert einer Kante für das Paar von Knoten ist, die einen Konnektivitätswert gleich oder höher als einen Konnektivitätsschwellenwert aufweisen, und gleich Null für die Knoten ist, die einen Konnektivitätswert niedriger als einen Konnektivitätsschwellenwert aufweisen;

e. Verwenden des Prozessors, um eine Adjazenzmatrix (10) zu definieren, die einen Adjazenzvektor für jeden Knoten enthält, wobei der Wert jedes Elements eines Adjazenzvektors dem Wert desselben Elements des äquivalenten Konnektivitätsvektors entspricht, aber auf 1 eingestellt wird, falls dieses Element größer als Null ist;

f. Einspeisen des Satzes von Konnektivitätsvektoren und der Adjazenzmatrix (10) als eine Eingabe in ein vom Prozessor ausgeführtes Graphenaufmerksamkeitsnetzwerk, wobei das Graphenaufmerksamkeitsnetzwerk dazu konfiguriert ist, eine Transformation über jeden Konnektivitätsvektor unter Verwendung der Adjazenzmatrix (10) als eine Eingabe anzuwenden, wodurch ein endgültiger Ausgabewert für jeden Konnektivitätsvektor erhalten wird;

g. Anordnen jedes endgültigen Ausgabewerts in einem zeitlichen Ausgabevektor, so dass ein zeitlicher Ausgabevektor für alle Knoten für das Zeitfenster erhalten wird;

h. Wiederholen der Schritte c. bis g. für jedes Zeitfenster, wodurch eine Anzahl von zeitlichen Ausgabevektoren (20) erhalten wird, die der Anzahl der Zeitfenster entspricht; und

i. Erhalten eines endgültigen Ausgabevektors durch Verwenden des Graphenaufmerksamkeitsnetzwerkes, um den Mittelwert der zeitlichen Ausgabevektoren (20) zu berechnen,

und wobei

das Graphenaufmerksamkeitsnetzwerk zuvor trainiert wurde, indem die Schritte b. bis i. mit einem Datensatz durchgeführt wurden, der Datendateien von Elektroenzephalographievorrichtungen umfasst, und indem die Ausgabe des Graphenaufmerksamkeitsnetzwerkes korrigiert wurde,

das Graphenaufmerksamkeitsnetzwerk eine erste Graphenaufmerksamkeitsschicht und eine zweite Graphenaufmerksamkeitsschicht umfasst, wobei

die erste Graphenaufmerksamkeitsschicht dazu konfiguriert ist, die Adjazenzmatrix und die Konnektivitätsvektoren als Eingaben zu empfangen, und zwei Aufmerksamkeitsmechanismen (13) umfasst, wobei jeder Aufmerksamkeitsmechanismus dazu konfiguriert ist, einen Ausgabevektor (14) mit vier Werten für jeden Konnektivitätsvektor bereitzustellen, so dass die erste Graphenaufmerksamkeitsschicht dazu konfiguriert ist, für jeden Konnektivitätsvektor einen ersten Ausgabevektor (15) mit acht Werten bereitzustellen, der durch die Verkettung der beiden Ausgabevektoren (14) mit vier Werten erhalten wird, die von jedem Aufmerksamkeitsmechanismus (13) erzeugt werden;

die zweite Graphenaufmerksamkeitsschicht dazu konfiguriert ist, den ersten Ausgabevektor (15) und die Adjazenzmatrix als Eingaben zu empfangen, und vier Aufmerksamkeitsmechanismen (16) umfasst, wobei jeder Aufmerksamkeitsmechanismus dazu konfiguriert ist, einen Ausgabewert (17) bereitzustellen, so dass die zweite Graphenaufmerksamkeitsschicht dazu konfiguriert ist, für jeden ersten Ausgabevektor einen einzelnen Mittelwert (18) bereitzustellen, der durch Berechnen des Mittelwerts der vier Ausgabewerte (17) erhalten wird, die von jedem Aufmerksamkeitsmechanismus (16) erzeugt werden;

das Graphenaufmerksamkeitsnetzwerk ferner dazu konfiguriert ist, eine Sigmoidtransformation auf den Mittelwert anzuwenden, wodurch der endgültige Ausgabewert (19) erhalten wird, der dem Konnektivitätsvektor für das entsprechende Zeitfenster zugeordnet ist;

wobei der Schritt des Trainierens des Graphenaufmerksamkeitsnetzwerkes die Ausgabe der zweiten Graphenaufmerksamkeitsschicht verwendet und eine Verlustfunktion als die binäre Kreuzentropie mit Logitverlust berechnet;

wobei jeder Aufmerksamkeitsmechanismus (13) der ersten Graphenaufmerksamkeitsschicht dazu konfiguriert ist, die folgende Transformation anzuwenden:

$$h_i^{(l+1)} = \sigma\left(\sum_{j \in N(i)} \alpha_{ij}^{(l)} W h_j^{(l)}\right)$$

wobei

$h_i^{(l+1)}$ die Darstellung des Knotens i ist, die als die Ausgabe der Aufmerksamkeitsschicht erhalten wird;

$h_j^{(l)}$ die Darstellung der Knoten ist, die dem Knoten i benachbart sind;

**W** die Gewichtsmatrix für die Merkmalstransformation ist und eine Größe von Nx4 aufweist, wobei N die Anzahl der Knoten ist;

$\sigma$ die Aktivierungsfunktion ist und gleich der Exponential Linear Unit (alpha: 1,0) ist

$\alpha_{ij}^{(l)}$ der Aufmerksamkeitskoeffizient des Knotens i mit dem Knoten j ist,

$$\alpha_{ij}^{(l)} = softmax_j\left(\sigma_a(a^T * [\, W h_i \,\|\, W h_j\,])\right),$$

$\sigma_a$ die Aktivierungsfunktion für den Aufmerksamkeitskoeffizienten ist und LeakyReLU (Schritt = 0,2) ist;

$Wh_i \| Wh_j$ die Verkettung der transformierten Darstellungen von Knoten i und Knoten j ist;

$a^T$ die Aufmerksamkeitsgewichtsmatrix ist und eine Größe von 1x8 aufweist,

wobei jeder Aufmerksamkeitsmechanismus (16) der zweiten Graphenaufmerksamkeitsschicht dazu konfiguriert ist, die folgende Transformation anzuwenden:

$$h_i^{(l+1)} = \sigma\left(\sum_{j \in N(i)} \alpha_{ij}^{(l)} W h_j^{(l)}\right)$$

wobei $h_i^{(l+1)}$

die Darstellung des Knotens i ist, die als die Ausgabe der Aufmerksamkeitsschicht erhalten wird;

$h_j^{(l)}$ die Darstellung der Knoten ist, die dem Knoten i benachbart sind;

**W** die Gewichtsmatrix und eine Größe von 8x1 aufweist;

$\sigma$ die Aktivierungsfunktion ist und gleich der Identitätsfunktion ist;

$\alpha_{ij}^{(l)}$ der Aufmerksamkeitskoeffizient des Knotens i mit dem Knoten j ist,

$$\alpha_{ij}^{(l)} = softmax_j\left(\sigma_a(a^T * [\, W h_i \,\|\, W h_j\,])\right),$$

$\sigma_a$ die Aktivierungsfunktion für den Aufmerksamkeitskoeffizienten ist und LeakyReLU (Schritt = 0,2) ist;

$Wh_i \| Wh_j$ die Verkettung der transformierten Darstellungen von Knoten i und Knoten j ist;

$a^T$ die Aufmerksamkeitsgewichtsmatrix ist und eine Größe von 1x2 aufweist.

2. Verfahren nach einem der vorhergehenden Ansprüche, wobei der Teilbereich der Sekunden 2 Sekunden beträgt.

3. Verfahren nach einem der vorhergehenden Ansprüche, wobei der Schritt des Erhaltens des Konnektivitätswertes für jede Kante in Schritt c. durchgeführt wird, indem die Kovarianz der Daten des Knotenpaares der entsprechenden Kante durch das Produkt der Standardabweichungen der Daten jedes der beiden Knoten des Knotenpaares geteilt wird.

4. Verfahren nach einem der vorhergehenden Ansprüche, wobei der Konnektivitätsschwellenwert zwischen 0,6 und 0,7 liegt.

**Revendications**

1. Procédé de traitement d'un fichier de données d'électroencéphalographie, le procédé comprenant les étapes consistant à :

   a. un dispositif d'électroencéphalographie (1) contenant des électrodes (2) produit un fichier de données contenant une pluralité de canaux, chaque canal contenant des données d'une électrode pour une plage temporelle, la plage temporelle étant la même pour chaque canal et durant une pluralité de secondes

   b. utiliser un processeur (5) pour récupérer le fichier de données et extraire au moins une fenêtre temporelle de la plage temporelle, chaque fenêtre temporelle durant une sous-plage de secondes ;

   c. utiliser le processeur pour générer un graphe pour l'une des fenêtres temporelles, en utilisant les données de tous les canaux dans la fenêtre temporelle correspondante, dans lequel le graphe contient des nœuds (7) et des arêtes (8), chaque arête (8) reliant une paire de nœuds (7), dans lequel chaque nœud (7) se rapporte à une électrode associée à un canal et dans lequel chaque arête (8) contient une valeur de connectivité obtenue à partir des données pour la paire de nœuds (7) correspondante reliée par l'arête (8) ;

   **caractérisé en ce que** le procédé comprend en outre les étapes consistant à :

   d. utiliser le processeur (5) pour définir un vecteur de connectivité pour chaque nœud, dans lequel le vecteur de connectivité a une taille de Nx1, N étant le nombre de nœuds, et dans lequel chaque élément du vecteur de connectivité est égal à la valeur de connectivité d'une arête pour la paire de nœuds ayant une valeur de connectivité égale ou supérieure à un seuil de connectivité, et est égal à zéro pour les nœuds ayant une valeur de connectivité inférieure à un seuil de connectivité ;

   e. utiliser le processeur pour définir une matrice d'adjacence (10) contenant un vecteur d'adjacence pour chaque nœud, dans lequel la valeur de chaque élément d'un vecteur d'adjacence correspond à la valeur du même élément du vecteur de connectivité équivalent, mais ajustée à 1 si cet élément est supérieur à zéro ;

   f. introduire l'ensemble des vecteurs de connectivité et la matrice d'adjacence (10) en tant qu'entrée dans un réseau d'attention de graphe exécuté par le processeur, dans lequel le réseau d'attention de graphe est configuré pour appliquer une transformation sur chaque vecteur de connectivité en utilisant la matrice d'adjacence (10) en tant qu'entrée, obtenant ainsi une valeur de sortie finale pour chaque vecteur de connectivité ;

   g. agencer chaque valeur de sortie finale dans un vecteur de sortie temporel, de sorte qu'un vecteur de sortie temporel soit obtenu pour tous les nœuds pour la fenêtre temporelle ;

   h. répéter les étapes c. à g. pour chaque fenêtre temporelle, obtenant ainsi un nombre de vecteurs de sortie temporels (20) correspondant au nombre de fenêtres temporelles ; et

   i. obtenir un vecteur de sortie final en utilisant le réseau d'attention de graphe pour calculer la valeur moyenne des vecteurs de sortie temporels (20),
   et dans lequel
   le réseau d'attention de graphe a été préalablement entraîné en exécutant les étapes b. à i. avec un ensemble de données comprenant des fichiers de données de dispositifs d'électroencéphalographie et en corrigeant la sortie du réseau d'attention de graphe,
   le réseau d'attention de graphe comprend une première couche d'attention de graphe et une seconde couche d'attention de graphe, dans lequel
   la première couche d'attention de graphe est configurée pour recevoir la matrice d'adjacence et les vecteurs de connectivité en tant qu'entrées et comprend deux mécanismes d'attention (13), chaque mécanisme d'attention étant configuré pour fournir un vecteur de sortie (14) de quatre valeurs pour chaque vecteur de connectivité, de sorte que la première couche d'attention de graphe est configurée pour fournir, pour chaque vecteur de connectivité, un premier vecteur de sortie (15) de huit valeurs obtenu par la concaténation des deux vecteurs de sortie (14) de quatre valeurs produits par chaque mécanisme d'attention (13) ;
   la seconde couche d'attention de graphe est configurée pour recevoir le premier vecteur de sortie (15) et la matrice d'adjacence en tant qu'entrées, et comprend quatre mécanismes d'attention (16), chaque mécanisme d'attention étant configuré pour fournir une valeur de sortie (17), de sorte que la seconde couche d'attention de graphe est configurée pour fournir, pour chaque premier vecteur de sortie, une unique valeur moyenne (18) obtenue en calculant la valeur moyenne des quatre valeurs de sortie (17) produites par chaque mécanisme d'attention (16) ;
   le réseau d'attention de graphe est en outre configuré pour appliquer une transformation sigmoïde à la valeur moyenne, obtenant ainsi la valeur de sortie finale (19) associée au vecteur de connectivité pour la fenêtre temporelle correspondante ;
   l'étape d'entraînement du réseau d'attention de graphe utilise la sortie de la seconde couche d'attention de graphe et calcule une fonction de perte sous la forme d'une entropie croisée binaire avec perte logit ;
chaque mécanisme d'attention (13) de la première couche d'attention de graphe est configuré pour appliquer la transformation suivante :

$$h_i^{(l+1)} = \sigma\left(\sum_{j \in N(i)} \alpha_{ij}^{(l)} W h_j^{(l)}\right)$$

dans lequel

$h_i^{(l+1)}$ est la représentation du nœud i obtenue en tant que sortie de la couche d'attention ;

$h_j^{(l)}$ est la représentation des nœuds voisins du nœud i ;

**W** est la matrice de pondération pour la transformation de caractéristiques et a une taille de Nx4, N étant le nombre de nœuds ;
$\sigma$ est la fonction d'activation et est égale à une Unité Linéaire Exponentielle (alpha : 1,0)

$\alpha_{ij}^{(l)}$ est le coefficient d'attention du nœud i avec le nœud j,

$$\alpha_{ij}^{(l)} = softmax_j\left(\sigma_a\big(a^T * [\,Wh_i \,||\, Wh_j\,]\big)\right),$$

$\sigma_a$ est la fonction d'activation du coefficient d'attention et est LeakyReLU (pas = 0,2) ;
$Wh_i \,||\, Wh_j$ est la concaténation des représentations transformées du nœud i et du nœud j ;
$a^T$ est la matrice de pondération d'attention et a une taille de 1x8.
chaque mécanisme d'attention (16) de la seconde couche d'attention de graphe est configuré pour appliquer la transformation suivante :

$$h_i^{(l+1)} = \sigma\left(\sum_{j \in N(i)} \alpha_{ij}^{(l)} W h_j^{(l)}\right)$$

dans lequel

$h_i^{(l+1)}$ est la représentation du nœud i obtenue en tant que sortie de la couche d'attention

$h_j^{(l)}$ est la représentation des nœuds voisins du nœud i ;

**W** est la matrice de pondération et a une taille de 8x1 ;
$\sigma$ est la fonction d'activation et est égale à la fonction identité ;

$\alpha_{ij}^{(l)}$ est le coefficient d'attention du nœud i avec le nœud j,

$$\alpha_{ij}^{(l)} = softmax_j\left(\sigma_a\big(a^T * [\,Wh_i \,||\, Wh_j\,]\big)\right),$$

$\sigma_a$ est la fonction d'activation du coefficient d'attention et est LeakyReLU (pas = 0,2) ;
$Wh_i \,||\, Wh_j$ est la concaténation des représentations transformées du nœud i et du nœud j ;
$a^T$ est la matrice de pondération d'attention et a une taille de 1x2.

2. Procédé selon l'une quelconque des revendications précédentes, dans lequel la sous-plage de secondes est de 2 secondes.

3. Procédé selon l'une quelconque des revendications précédentes, dans lequel l'étape d'obtention de la valeur de connectivité pour chaque arête à l'étape c. est effectuée en divisant la covariance des données de la paire de nœuds de l'arête correspondante par le produit des écarts-types des données de chacun des deux nœuds de la paire de nœuds.

4. Procédé selon l'une quelconque des revendications précédentes, dans lequel le seuil de connectivité est compris entre 0,6 et 0,7.

**FIG. 1**

**FIG. 2**

**FIG. 3**

| | | | | | | | |
|---|---|---|---|---|---|---|---|
| 1 | 0,86 | 0,78 | 0,64 | 0,32 | 0,91 | 0,54 | 0,22 |
| 0,86 | 1 | 0,58 | 0,98 | 0,61 | 0,49 | 0,85 | 0,33 |
| 0,78 | 0,58 | 1 | 0,78 | 0,67 | 0,79 | 0,39 | 0,51 |
| 0,64 | 0,98 | 0,78 | 1 | 0,81 | 0,54 | 0,84 | 0,73 |
| 0,32 | 0,61 | 0,67 | 0,81 | 1 | 0,92 | 0,51 | 0,78 |
| 0,91 | 0,49 | 0,79 | 0,54 | 0,92 | 1 | 0,49 | 0,82 |
| 0,54 | 0,85 | 0,39 | 0,84 | 0,51 | 0,49 | 1 | 0,86 |
| 0,22 | 0,33 | 0,51 | 0,73 | 0,78 | 0,82 | 0,86 | 1 |

| | | | | | | | |
|---|---|---|---|---|---|---|---|
| 1 | 1 | 1 | 0 | 0 | 1 | 0 | 0 |
| 1 | 1 | 0 | 1 | 0 | 0 | 1 | 0 |
| 1 | 0 | 1 | 1 | 0 | 1 | 0 | 0 |
| 0 | 1 | 1 | 1 | 1 | 0 | 1 | 1 |
| 0 | 0 | 0 | 1 | 1 | 1 | 0 | 1 |
| 1 | 0 | 1 | 0 | 1 | 1 | 0 | 1 |
| 0 | 1 | 0 | 1 | 0 | 0 | 1 | 1 |
| 0 | 0 | 0 | 1 | 1 | 1 | 1 | 1 |

**FIG. 4**

**FIG. 5**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **JIANCHAO, LU et al.** *PearNet: A Pearson Correlation-based Graph Attention Network for Sleep Stage Recognition*

- **KLEPL, DOMINIK et al.** *Adaptive Gated Graph Convolutional Network for Explainable Diagnosis of Alzheimer's Disease Using EEG Data*